# FASCICULE DE BREVET EUROPEEN

(11) **EP 3 595 732 B1**
(45) Date de publication et mention de la délivrance du brevet: **25.08.2021**
(21) Numéro de dépôt: 18712840.0
(22) Date de dépôt: 15.03.2018
(51) Int. Cl.: A61L 9/013

(54) **UTILISATION D'UNE COMPOSITION RENFERMANT DU 1,8-PARA-MENTHÈNETHIOL ET DE L'ACÉTATE DE 3-MERCAPTOHEXYLE COMME MASQUEUR D'ODEURS**
VERWENDUNG EINER ZUSAMMENSETZUNG MIT 1,8-PARA-MENTHENTHIOL UND 3-MERCAPTOHEXYLACETAT ALS GERUCHSMASKIERER
USE OF A COMPOSITION CONTAINING 1,8-PARA-MENTHENETHIOL AND 3-MERCAPTOHEXYL ACETATE AS AN ODOUR-MASKING AGENT

(30) Priorité: 17.03.2017 FR 1752175
(43) Date de publication de la demande: 22.01.2020
(73) Titulaire: Jafer Enterprises R&D SL, 08403 Granollers (ES)
(72) Inventeur: DELMAS, Thomas, 06600 Antibes (FR); GOUTEYRON, Antoine, 06110 Le Cannet (FR); PEREZ, Marion, 06270 Villeneuve-Loubet (FR)
(74) Mandataire: Cabinet Becker et Associés
(86) Numéro de dépôt international: PCT/EP2018/056519
(87) Numéro de publication internationale: WO 2018/167206

(56) Documents cités:
- WO-A1-2016/138186
- US-A1- 2015 030 744
- US-A1- 2016 354 304
- Thokchom Prasanta Singh ET AL: "Phytochemical and pharmacological profile of Zanthoxylum armatum DC.  An overview", Indian Journal of Natural Products and Resources, 30 septembre 2011 (2011-09-30), pages 275-285, XP055394561, Extrait de l'Internet: URL:http://beauty-review.nl/wp-content/upl oads/2014/06/Phytochemical-and-pharmacolog ical-profile-of-Zanthoxylum-armatum-DC.-An -overview.pdf [extrait le 2017-07-28] cité dans la demande

## Description

### OBJET DE L'INVENTION

La présente invention concerne l'utilisation d'une composition renfermant du 1,8-para-menthènethiol et de l'acétate de 3-mercaptohexyle, telle qu'un extrait végétal et en particulier un extrait de Timur (*Zanthoxylum armatum*), comme masqueur d'odeurs. Elle concerne également un produit désodorisant sous forme d'aérosol, de bougie ou de diffuseur de parfum à mèche ou électrique, contenant cette composition.

### ARRIERE-PLAN DE L'INVENTION

De nombreux produits capables de réduire ou masquer les mauvaises odeurs sont disponibles dans le commerce et ont été décrits dans la littérature. Ces produits désodorisants agissent soit par voie physique, en piégeant les molécules responsables des mauvaises odeurs dans des filtres ayant une porosité et une surface spécifique adaptées, notamment des cyclodextrines, soit par voie chimique, en modifiant chimiquement ces molécules, soit par voie sensorielle, en masquant les mauvaises odeurs à l'aide de parfums qui permettent de réduire la perception des mauvaises odeurs. Parmi ces derniers, la plupart se présentent sous forme d'aérosols utilisant des gaz propulseurs à base d'hydrocarbures, qui permettent une diffusion très rapide d'un parfum renfermant un mélange d'aldéhydes. Si ces composés sont capables de réduire l'impact des mauvaises odeurs, ils sont sujets à l'oxydation et provoquent, après vaporisation sur des tissus, un jaunissement prononcé à la lumière.

Des recherches ont donc été entreprises pour identifier d'autres composés capables de masquer efficacement les mauvaises odeurs.

Dans ce contexte, il est connu que le 1,8-para-menthènethiol ou thioterpinéol peut être utilisé comme masqueur d'odeurs (WO 2016/058710). Toutefois, il est apparu à la Demanderesse que son efficacité n'était pas toujours suffisante à l'encontre de certaines odeurs et, après de nombreuses recherches, il a été mis en évidence que l'efficacité de ce composé pouvait être renforcée en l'associant à l'acétate de 3-mercaptohexyle. Ce dernier est certes connu comme molécule odorante responsable notamment des arômes de fruits exotiques de certains fruits, mais il n'était pas prévisible qu'il permette d'améliorer l'efficacité du thioterpinéol, dans la mesure où il est bien connu que la combinaison de molécules odorantes peut présenter un effet antagoniste de celui obtenu lorsque ces molécules sont utilisées individuellement.

La Demanderesse a par ailleurs démontré que la combinaison de molécules précitée était présente dans certains extraits végétaux et en particulier dans une composition volatile extraite de *Zanthoxylum armatum,* ce qui n'était pas connu jusqu'alors (voir notamment T.P. Singh et al., Indian Journal of Natural Products and Resources, 30 septembre 2011, pages 275-285). La Demanderesse a ainsi envisagé l'utilisation de ces extraits comme agents masqueurs d'odeurs dans différents produits désodorisants.

L'espèce *Zanthoxylum armatum* ou *planispinum* appartient à la famille des Rutacées. Il s'agit d'un végétal qui croît en Asie et en particulier au Népal, dont les baies constituent le poivre de Timut. Il a été suggéré d'utiliser des extraits de Timur (sans précision de leur composition ou de leur mode d'extraction) comme agent générant des picotements dans des compositions aromatisantes (US 2015/030744) ou parfumantes (WO 2016/138186). Il a également été mentionné un effet déodorant et antiseptique d'extraits de Timur riches en flavonoïdes (voir *supra,* T.P. Singh et al). Ce type d'extrait est habituellement obtenu par extraction hydro-alcoolique. De son côté, l'huile essentielle extraite de ces baies est connue pour traiter de nombreuses pathologies. Il a également été envisagé d'utiliser le produit d'extraction à l'aide de CO₂ supercritique des baies de Timur dans des produits pour les lèvres (US 2016/354304). Toutefois, à la connaissance de la Demanderesse, il n'a encore jamais été suggéré que ce type d'extrait pouvait présenter un intérêt comme masqueur d'odeurs.

### RESUME DE L'INVENTION

L'invention a ainsi pour objet l'utilisation d'une composition renfermant du 1,8-para-menthènethiol et de l'acétate de 3-mercaptohexyle comme masqueur d'odeurs.

Elle a également pour objet un produit désodorisant sous forme d'aérosol, de bougie ou de diffuseur de parfum à mèche ou électrique, contenant la composition précitée.

### DESCRIPTION DETAILLEE

La composition utilisée selon l'invention comprend un thiol et un ester particuliers, à savoir le 1,8-para-menthènethiol et l'acétate de 3-mercaptohexyle. Elle renferme en outre avantageusement au moins un constituant choisi parmi : le linalool, le limonène, le myrcène, le cinnamate de méthyle, le β-phellandrène, le 4-mercapto-4-méthyl-pentan-2-one, le 3-mercaptohexanol et un mélange de ceux-ci, de préférence un mélange de tous les constituants précités. Selon une forme d'exécution de l'invention, la composition renferme de 5 à 99% de produits volatils et semi-volatils qui contiennent de 50 à 95% en poids d'une combinaison de linalool, limonène, myrcène et cinnamate de méthyle. Par « produit volatil », on entend un produit possédant une pression de vapeur inférieure à 13,34 Pa (0,1 mm Hg) à 20°C ou un point d'ébullition inférieur à 216°C. Par « produit semi-volatil », on entend un produit possédant un point d'ébullition compris entre 216 et 350°C.

L'ensemble des constituants précités peuvent être issus d'un ou plusieurs extraits végétaux qui les contiennent à l'état naturel. Selon une forme d'exécution préférée de l'invention, la composition est donc un extrait végétal ou un mélange d'extraits végétaux. Selon une autre forme d'exécution, les constituants de la composition selon l'invention peuvent être chacun obtenus indépendamment par synthèse chimique ou à partir d'un extrait végétal, puis mélangés pour former ladite composition.

Lorsque la composition utilisée selon l'invention est un extrait végétal, on préfère qu'il s'agisse d'un extrait de Timur (*Zanthoxylum armatum*), qui peut être obtenu à partir de toute partie du Timur et notamment de ses feuilles, de son tronc, de ses fruits (baies) ou de leur péricarpe. Plus, préférentiellement, on utilise un extrait de baies de Timur.

Dans un mode de réalisation préféré de l'invention, les baies de Timur peuvent être séchées et/ou broyées par tout moyen préalablement à leur extraction, notamment par cryobroyage. Il a en effet été observé que les baies broyées présentaient un meilleur équilibre entre leurs différentes notes olfactives, dû notamment à une modification du ratio monoterpènes / linalool. La granulométrie moyenne (D50) des baies broyées peut notamment être comprise entre 200 et 600µm, telle que mesurée par tamisage.

Les composés utilisés selon l'invention étant également présents séparément dans d'autres extraits végétaux que ceux issus du Timur, la composition selon l'invention pourra en variante être constituée d'un mélange d'extraits végétaux, par exemple d'un mélange d'extrait de raisin noir, de fruit de la passion ou de houblon (contenant de l'acétate de 3-mercaptohexyle) et de pamplemousse, de citronnier épineux (*Poncirus trifoliata*) ou d'orange (contenant du 1,8-para-menthènethiol).

L'extraction des parties de plante choisies pour obtenir le ou les extraits végétaux utilisés selon l'invention peut être effectuée par hydrodistillation ou préférentiellement à l'aide de CO₂ supercritique. Dans le cas du Timur, l'extraction au CO₂ supercritique permet d'obtenir un extrait présentant un parfum plus puissant, plus durable et moins riche en aldéhydes que l'extraction par hydrodistillation. Ces caractéristiques pourraient être liées à la plus grande richesse en acides gras et en esters de la fraction volatile ainsi obtenue. Les conditions de mise en œuvre de ces procédés pourront être facilement déterminées par l'homme de l'art. Ainsi, l'extraction au CO₂ supercritique peut par exemple être effectuée à une température de 30 à 60°C, par exemple de 35 à 50°C, sous une pression de 50 à 150 bars, par exemple de 80 à 100 bars.

L'extrait végétal obtenu peut éventuellement être séché par tout moyen, généralement jusqu'à une teneur en humidité de 0 à 10% en poids, avant d'être utilisé selon l'invention.

La présence de 1,8-para-menthènethiol et d'acétate de 3-mercaptohexyle dans l'extrait végétal peut être confirmée notamment par modification de ces thiols avec la 4,4'-dithiodipyridine ou le N-phénylmaléimide, respectivement, puis analyse par chromatographie en phase liquide à ultra haute performance (UHPLC) couplée à une analyse qualitative et quantitative par spectrométrie de masse (MS QqQ).

La composition selon l'invention peut être utilisée pour masquer tout type d'odeurs, notamment choisies parmi des odeurs indolées, aminées (en particulier l'ammoniac et le skatole), thiolées (notamment le trisulfure de diméthyle ou le thiophénol) ou acides (notamment l'acide isovalérique). Elle peut par ailleurs servir à masquer ces odeurs dans tout type d'environnement dans lequel elle est appliquée, notamment sur le corps, dans des locaux à usage domestique, commercial ou industriel. En variante, cette composition peut être combinée à des produits malodorants en eux-mêmes ou susceptibles de générer de mauvaises odeurs lors de leur utilisation. La composition selon l'invention peut ainsi être ajoutée à un produit désodorisant ; un produit détergent tel qu'une lessive ou un nettoyant ménager (notamment un produit de nettoyage des toilettes ou de la vaisselle) ; un produit d'hygiène corporelle tel qu'un déodorant, un anti-transpirant, un savon ou un shampooing ; ou un produit de coloration capillaire, sans que cette liste ne soit limitative. Dans une forme d'exécution préférée de l'invention, cette composition est intégrée à un produit désodorisant. L'invention concerne donc également un produit désodorisant sous forme d'aérosol, de bougie ou de diffuseur de parfum à mèche ou électrique, contenant une composition renfermant du 1,8-para-menthènethiol et de l'acétate de 3-mercaptohexyle, telle que définie précédemment. Ce produit est en particulier destiné à être utilisé comme désodorisant d'intérieur et/ou de textiles.

Outre la composition précitée, le produit qui l'intègre peut comprendre différents constituants permettant notamment à ce produit de se présenter sous une forme adaptée à son usage, notamment sous forme de liquide, de gel, de mousse ou de crème ou encore sous forme solide. Ces constituants peuvent par exemple comprendre des solvants tels que de l'eau et/ou des solvants organiques polaires ou apolaires, hydrocarbonés ou siliconés ; des agents tensioactifs ; des gélifiants de phase aqueuse ; des épaississants de phase huileuse ; des conservateurs ; des antioxydants ; des agents chélatants ; des polyols ; des absorbeurs d'odeurs tels que des zéolithes, des cyclodextrines, de la silice, des aluminosilicates ou du charbon actif ; des absorbeurs UV ; des agents antimicrobiens ; des parfums tels que des huiles essentielles ; des pigments ; et des colorants, sans que cette liste ne soit limitative, pour autant que ces constituants ne nuisent pas aux propriétés désodorisantes de la composition selon l'invention. La composition selon l'invention peut par ailleurs être véhiculée dans des microsphères, des nanocapsules, des microcapsules, des liposomes ou tout autre vecteur permettant son incorporation dans le produit précité et éventuellement la libération prolongée de ses constituants. L'incorporation de la composition selon l'invention aux produits précités peut notamment se faire par mélange de leurs constituants.

La composition selon l'invention représente par exemple de 0,01 à 20% en poids, de préférence de 0,05 à 5% en poids, par rapport au poids du produit auquel elle est incorporée.

Selon un mode de réalisation de l'invention, la composition peut être imprégnée sur un produit non-tissé, notamment une lingette. Dans un autre mode de réalisation encore, la composition selon l'invention peut être incorporée à un produit d'hygiène absorbant, tel qu'une couche pour bébé, et en particulier dans le noyau absorbant et/ou l'une au moins des couches non tissées et/ou des films plastiques constituant les couches supérieure et inférieure du produit absorbant et/ou au sein d'élastiques.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Préparation d'une composition selon l'invention

On a préparé un réducteur de mauvaises odeurs (MOR) constitué d'un extrait de baies de Timur obtenu par extraction à l'aide de CO₂ supercritique.

Pour ce faire, des baies de Timur sont d'abord cryobroyées, en les traitant par un flux d'azote à -196°C avant de les passer dans un broyeur à couteaux (modèle SM100® de RETSCH). On obtient ainsi une poudre homogène dont la granulométrie se situe majoritairement dans la plage de 200 à 600µm. Cette poudre est placée dans une cartouche en acier inoxydable et cette cartouche est placée dans un extracteur à fluide supercritique (SFE5® de SEPAREX). L'extraction est effectuée à l'aide de CO₂ supercritique, dans un ratio solvant / matière première d'environ 4. La pression au sein de l'extracteur est réglée à 80-120 bars et la température à 35-50°C.

L'analyse de l'extrait obtenu montre qu'il renferme du 1,8-para-menthènethiol et de l'acétate de 3-mercaptohexyle.

### Exemple 2 : Effet désodorisant d'une composition à base de Zanthoxylum armatum

### Exemple 2A: Test sur cotons

On a réalisé une série de tests visant à déterminer l'effet masqueur d'odeur du MOR de l'Exemple 1 vis-à-vis de différentes molécules responsables de mauvaises odeurs (MO). Pour ce faire, un ou deux carrés de coton de 1 cm², imprégnés de différentes concentrations de MO, ont été placés dans des flacons scellés de 15 mL, en présence de deux concentrations différentes du MOR. L'intensité olfactive de la mauvaise odeur a été évaluée par un panel de deux experts, 2h30 après avoir scellé le flacon, et elle a été comparée à un témoin constitué d'un flacon identique mais ne comprenant pas de MOR.

Les résultats obtenus sont rassemblés dans le Tableau 1 ci-dessous, où le pourcentage de réduction de mauvaise odeur correspond à la fraction de la mauvaise odeur du témoin encore présente dans le flacon testé et "MOR x A" désigne une quantité de MOR qui est A fois supérieure à celle de MO.

**Tableau 1**

| MO | Nb cotons | Réduction mauvaise odeur (%) du Timur CO₂ | | |
|---|---|---|---|---|
| | | MOR x 40 | MOR x 100 | MOR x 200 |
| Ammoniac | 2 | 100 | 100 | 100 |
| Acide isovalérique | 1 | 100 | 100 | 100 |
| Thiophénol | 1 | 100 | 100 | 100 |

L'extrait de Timur selon l'invention présente donc une grande efficacité contre les mauvaises odeurs testées.

### Exemple 2B : Test en conditions réelles

Cet effet a été confirmé à plus grande échelle. Pour cela, on a vaporisé une solution modèle de MO dans une pièce neutre normalisée de 25 m³, dont la température était régulée à 22 ± 2°C. La solution utilisée renfermait un mélange de 5 à 100 ppm de skatole, de 5 à 100 ppm de trisulfure de diméthyle, de 5 à 100 ppm de thiophénol, de 80 à 4000 ppm d'ammoniac et de 30 à 600 ppm d'acide isovalérique. Dix pulvérisations ont été effectuées. Une bougie formulée à l'aide du MOR de l'Exemple 1 a alors été allumée. Elle contenait de 1 à 10% en poids d'extrait de Timur et de 90 à 99% en poids d'un mélange pré-formulé de cire d'abeille (33%) et d'acide stéarique (66%). L'intensité olfactive dans le temps de la MO a été évaluée par un panel de deux experts et cinq naïfs, en vue de déterminer le temps au bout duquel la MO n'était plus perceptible. Une comparaison a été effectuée dans les mêmes conditions mais sans bougie, et avec une bougie dépourvue de MOR selon l'invention (pour évaluer l'effet de la combustion).

Les résultats obtenus sont rassemblés dans le Tableau 2 ci-dessous.

**Tableau 2**

| Bougie testée | -- | Neutre | 1% MOR | 3% MOR | 5% MOR | 10% MOR |
|---|---|---|---|---|---|---|
| Temps d'élimination de MO | 55 min | 40 min | 30 min | 25 min | 20 min | 15 min |

Ces résultats confirment l'efficacité de la composition selon l'invention.

### Exemple 3 : Mise en évidence d'un effet promoteur de réduction des mauvaises odeurs

L'Exemple 2A a été répété en remplaçant l'extrait de Timur utilisé par une solution de 1,8-para-menthènethiol (PMT), seul ou combiné à de l'acétate de 3-mercapto hexyle (3MHA) dans un ratio pondéral de 50/50 pour conserver un rapport molaire [MOR]/[MO] de 0,2. Cette solution renfermait 0,025% du mélange 3MHA/PMT et 99,975% d'éthanol.

Les résultats obtenus sont rassemblés dans le Tableau 3 ci-dessous.

**Tableau 3**

| | Réduction mauvaise odeur (%) | | | |
|---|---|---|---|---|
| MO | Ammoniac | Acide isovalérique | Thiophénol | Skatole |
| PMT | 0 | 40 | 100 | 100 |
| PMT + 3MHA (50/50) | 100 | 100 | 100 | 100 |
| 3MHA | 80 | 100 | 60 | 100 |

Cet essai démontre que le 3MHA a un effet promoteur de la réduction des mauvaises odeurs dues à l'acide isovalérique et qu'un effet synergique entre le 3MHA et le PMT est même obtenu dans le cas de l'ammoniac. En outre, le 3MHA n'affecte pas négativement l'effet de réduction des autres mauvaises odeurs, obtenu avec le PMT seul.

### Exemple 4 : Produits incorporant la composition selon l'invention

Les formulations suivantes ont été préparées de manière classique pour l'homme de l'art, en mélangeant les ingrédients ci-dessous dans les proportions pondérales indiquées.

| Ingrédient | Formule 4.1.1 | Formule 4.1.2 | Cas général |
|---|---|---|---|
| Eau désionisée | qsp 100% | qsp 100% | qsp 100% |
| Polyéthylèneimine | 0,0650% | 0,0650% | 0-1% |
| Diéthylène Glycol | 0,175% | 0,175% | 0-0,2% |
| Acide citrique | 0,05% | 0,05% | 0-0,2% |
| 1,2-Benzisothiazolin-3-one | 0,003% | 0,003% | 0-0,01 |
| Ethanol | 3% | 3% | 0-5% |
| Hydroxyde de sodium | 0,003% | 0,003% | 0,001-0,01% |
| Parfum | 0% | 0,4% | 0-2% |
| Extrait de Timur selon l'Exemple 1 | 0,05% | 1% | 0,05-20% |

### Exemple 4.1 : Désodorisants d'intérieur et de textiles

### Exemple 4.2 : Aérosol désodorisant

| Ingrédient | Exemple | Cas général |
|---|---|---|
| Eau désionisée | qsp 100% | qsp 100% |
| Acide polyacrylique | 0,195% | 0-0,4% |
| Citrate de sodium | 0,05% | 0-0,2% |
| Acide citrique | 0,1% | 0-0,2% |
| Sodium lauryl sulfate | 0,145% | 0-0,2% |
| Ethanol | 5% | 0,1-10% |
| Hydroxyde de sodium | 0,075% | 0-0,1% |
| Organosilicone | 0,2% | 0-0,2% |
| PEG 60 | 1,4% | 0-2% |
| Parfum | 1,2% | 0-1,5% |
| Extrait de Timur selon l'Exemple 1 | 4% | 0,05-20% |

### Exemple 4.3 : Déodorant

| Ingrédient | |
|---|---|
| Dipropylène glycol | 20-50% |
| Propylène glycol | 15-25% |
| Tripropylène glycol | 0-25% |
| Glycérine | 0-10% |
| PEG -8 | 0-20% |
| Eau | 0-qsp |
| Myristyl éther de propylène glycol | 0-2% |
| Ethanol | qsp 100% |
| Stéarate de sodium | 5-6% |
| EDTA | 0,05-0,5% |
| Parfum | 0-1,5% |
| Extrait de Timur selon l'Exemple 1 | 0,05-5% |

### Exemple 4.4 : Bougies

| Ingrédient | Formule 4.4.1 | Formule 4.4.2 |
|---|---|---|
| Type de bougie | Cierge | Pot |
| Cire de paraffine type 6003 | 5-50% | - |
| Acide stéarique | 5-50% | - |
| Polymère (Vybar®) | 5-50% | - |
| Alphawax® Jarisma 1218 | - | 5-50% |
| Alphawax® LA0186-T02 | - | 5-50% |
| Cire d'abeille blanche | - | 5-50% |
| Cire de Candelila | - | 5-50% |
| Parfum | 0-5% | 0-5% |
| Extrait de Timur selon l'Exemple 1 | 0,05-20% | 0,05-20% |
| Total | 100% | 100% |

### Exemple 4.5 : Cire fondante

| Ingrédient | |
|---|---|
| Triglycérides caprylique / caprique | 5-15% |
| Huile de soja | 5-15% |
| Polyisobutène hydrogéné | 5-15% |
| Lanoline | 5-15% |
| Cire d'abeille | 5-15% |
| Polyéthylène 400 | 5-15% |
| Cire microcristalline | 5-15% |
| Alpha-tocophérol | 0,05-0,2% |
| Parfum | 0-1,5% |
| Extrait de Timur selon l'Exemple 1 | 0,05-3% |
| Total | 100% |

### Exemple 4.6 : Composition pour diffuseur à mèche

| Ingrédient | |
|---|---|
| Ethanol | 60-80% |
| Eau désionisée | 0,05-5% |
| Parfum | 0-20% |
| Extrait de Timur selon l'Exemple 1 | 0,05-20% |
| Total | 100% |

### Exemple 4.7 : Composition pour diffuseur électrique

| Ingrédient | |
|---|---|
| Isopropyl myristate | 60-80% |
| Eau désionisée | 0,05-5% |
| Parfum | 0-20% |
| Extrait de Timur selon l'Exemple 1 | 0,05-20% |
| Total | 100% |

### Exemple 4.8 : Composition de coloration capillaire

| Ingrédient | |
|---|---|
| Peroxyde d'hydrogène | 0,2-0,7% |
| Acide peracétique | 0,5-2% |
| 1 para-phénylène diamine | 0,1-0,5% |
| Colorant Basic Red 76 | 0-0,1% |
| EDTA | 0,05-0,2% |
| Ceteareth-25 | 0,5-3% |
| Cocamidopropyl bétaïne | 0,5-3% |
| Alcool cétylique | 1-5% |
| Alcool stéarylique | 1-5% |
| Sulfite de sodium | 0,05-0,2% |
| Eau | qsp 100% |
| Extrait de Timur selon l'Exemple 1 | 0,01-2% |

Les formulations 4.1 à 4.8, utilisées dans les conditions usuelles, présentent l'avantage de masquer les odeurs issues des produits eux-mêmes (cas de l'ammoniaque pour l'exemple 4.8) ou bien les odeurs issues du milieu ambiant (cas des diffuseurs ou bougies) ou encore des odeurs présentes après application (cas du déodorant 4.3).

## Revendications

1. Utilisation d'une composition renfermant du 1,8-para-menthènethiol et de l'acétate de 3-mercaptohexyle comme masqueur d'odeurs.

2. Utilisation selon la revendication 1, **caractérisée en ce que** ladite composition renferme en outre au moins un constituant choisi parmi : le linalool, le limonène, le myrcène, le cinnamate de méthyle, le β-phellandrène, le 4-mercapto-4-méthyl-pentan-2-one, le 3-mercaptohexanol et un mélange de ceux-ci, de préférence un mélange de tous les constituants précités.

3. Utilisation selon la revendication 2, **caractérisée en ce que** ladite composition renferme de 5 à 99% de produits volatils et semi-volatils qui contiennent de 50 à 95% en poids d'une combinaison de linalool, limonène, myrcène, cinnamate de méthyle et beta-phellandrène, les produits volatils possédant une pression de vapeur inférieure à 13,34 Pa (0,1 mm Hg) à 20°C ou un point d'ébullition inférieur à 216°C et lesdits produits semi-volatils possédant un point d'ébullition compris entre 216 et 350°C.

4. Utilisation selon l'une quelconque des revendications 1 à 3, **caractérisée en ce que** ladite composition est un extrait végétal ou un mélange d'extraits végétaux.

5. Utilisation selon la revendication 4, **caractérisée en ce que** ledit extrait végétal est obtenu par extraction à l'aide de CO₂ supercritique ou par hydrodistillation, de préférence à l'aide de CO₂ supercritique, d'une partie de plante.

6. Utilisation selon la revendication 4 ou 5, **caractérisée en ce que** l'extrait végétal est un extrait de Timur (*Zanthoxylum armatum*).

7. Utilisation selon la revendication 6, **caractérisée en ce que** les baies de Timur sont broyées avant extraction.

8. Utilisation selon l'une quelconque des revendications 1 à 7, **caractérisée en ce que** lesdites odeurs sont choisies parmi des odeurs indolées, aminées, thiolées ou acides.

9. Utilisation selon l'une quelconque des revendications 1 à 8, **caractérisée en ce que** ladite composition est ajoutée à un produit désodorisant ; un produit détergent tel qu'une lessive ou un nettoyant ménager ; un produit d'hygiène corporelle tel qu'un déodorant, un anti-transpirant, un savon ou un shampooing ; un produit d'hygiène absorbant tel qu'une couche pour bébé ; ou un produit de coloration capillaire.

10. Produit désodorisant sous forme d'aérosol, de bougie ou de diffuseur de parfum à mèche ou électrique, contenant une composition renfermant du 1,8-para-menthènethiol et de l'acétate de 3-mercaptohexyle, telle que définie dans l'une quelconque des revendications 1 à 7.

## Patentansprüche

1. Verwendung einer Zusammensetzung, umfassend 1,8-para-Menthenthiol und 3-Mercaptohexylacetat als Geruchsmaskierer.

2. Verwendung gemäß Anspruch 1, **dadurch gekennzeichnet, dass** die Zusammensetzung außerdem wenigstens einen Bestandteil umfasst, ausgewählt aus Linalool, Limonen, Myrcen, Zimtsäuremethylester, β-Phellandren, 4-Mercapto-4-methylpentan-2-on, 3-Mercaptohexanol und einem Gemisch davon, vorzugsweise einem Gemisch aller vorgenannten Bestandteile.

3. Verwendung gemäß Anspruch 2, **dadurch gekennzeichnet, dass** die Zusammensetzung 5 bis 99% flüchtige und halbflüchtige Produkte umfasst, die 50 bis 99 Gew.-% einer Kombination aus Linalool, Limonen, Myrcen, Zimtsäuremethylester und β-Phellandren enthalten, wobei die flüchtigen Produkte einen Dampfdruck unter 13,34 Pa (0,1 mm Hg) bei 20°C oder einen Siedepunkt unter 216°C besitzen und die halbflüchtigen Produkte einen Siedepunkt zwischen 216 und 350°C besitzen.

4. Verwendung gemäß einem der Ansprüche 1 bis 3, **dadurch gekennzeichnet, dass** die Zusammensetzung ein Pflanzenextrakt oder ein Gemisch von Pflanzenextrakten ist.

5. Verwendung gemäß Anspruch 4, **dadurch gekennzeichnet, dass** der Pflanzenextrakt durch Extraktion mithilfe von überkritischem CO₂ oder durch Wasserdampfdestillation, vorzugsweise mithilfe von überkritischem CO₂, aus einem Pflanzenteil erhalten ist.

6. Verwendung gemäß Anspruch 4 oder 5, **dadurch gekennzeichnet, dass** der Pflanzenextrakt ein Extrakt aus Timur (*Zanthoxylum armatum*) ist.

7. Verwendung gemäß Anspruch 6, **dadurch gekennzeichnet, dass** die Beeren von Timur vor Extraktion zermahlen werden.

8. Verwendung gemäß einem der Ansprüche 1 bis 7, **dadurch gekennzeichnet, dass** die Gerüche aus indolartigen, aminartigen, thiolartigen oder säureartigen Gerüchen ausgewählt sind.

9. Verwendung gemäß einem der Ansprüche 1 bis 8, **dadurch gekennzeichnet, dass** die Zusammensetzung einem desodorierenden Produkt; einer Reinigungsmittelzusammensetzung wie einem Waschmittel oder einem Haushaltsreiniger; einem Körperpflegeprodukt wie einem Deodorant, einem Antitranspirant, einer Seife oder einem Shampoo; einem absorbierenden Hygieneprodukt wie einer Babywindel; oder einem Haarfärbemittel zugesetzt wird.

10. Desodorierendes Produkt in Form eines Aerosols, einer Kerze oder eines elektrischen oder Docht enthaltenden Duftstoffspenders, enthaltend eine Zusammensetzung, umfassend 1,8-para-Menthenthiol und 3-Mercaptohexylacetat, wie in einem der Ansprüche 1 bis 7 definiert.

## Claims

1. Use of a composition containing 1,8-para-menthenethiol and 3-mercaptohexyl acetate as an odor-masking agent.

2. Use according to Claim 1, **characterized in that** said composition also contains at least one constituent chosen from: linalool, limonene, myrcene, methyl cinnamate, β-phellandrene, 4-mercapto-4-methylpentan-2-one, 3-mercaptohexanol and a mixture thereof, preferably a mixture of all the abovementioned constituents.

3. Use according to Claim 2, **characterized in that** said composition contains from 5 to 99% of volatile and semi-volatile products which contain from 50 to 95% by weight of a combination of linalool, limonene, myrcene, methyl cinnamate and beta-phellandrene, the volatile products having a vapor pressure of less than 13.34 Pa (0.1 mm Hg) at 20°C or a boiling point of less than 216°C and said semi-volatile products having a boiling point of between 216 and 350°C.

4. Use according to any one of Claims 1 to 3, **characterized in that** said composition is a plant extract or a mixture of plant extracts.

5. Use according to Claim 4, **characterized in that** said plant extract is obtained by extraction using supercritical CO₂ or by hydrodistillation, preferably using supercritical CO₂, of a plant part.

6. Use according to Claim 4 or 5, **characterized in that** the plant extract is an extract of timur (*Zanthoxylum armatum*).

7. Use according to Claim 6, **characterized in that** the timur berries are ground before extraction.

8. Use according to any one of Claims 1 to 7, **characterized in that** said odors are chosen from indole, amine, thiol or acid odors.

9. Use according to any one of Claims 1 to 8, **characterized in that** said composition is added to a deodorizing product; a detergent product such as laundry detergent or a household cleaning product; a bodily hygiene product such as a deodorant, an antiperspirant, a soap or a shampoo; an absorbent hygiene product such as a baby's diaper; or a hair dyeing product.

10. Deodorizing product in the form of an aerosol, candle, or electrical or wick fragrance diffuser, containing a composition containing 1,8-para-menthenethiol and 3-mercaptohexyl acetate, as defined in any one of Claims 1 to 7.
